# EUROPEAN PATENT SPECIFICATION

(11) **EP 3 435 840 B1**
(45) Date of publication and mention of the grant of the patent: **04.12.2019**
(21) Application number: 17713692.6
(22) Date of filing: 30.03.2017
(51) Int. Cl.: A61B 5/00, A61B 5/11

(54) **DEVICE, SYSTEM AND COMPUTER PROGRAM PRODUCT FOR DETECTING MUSCLE SEIZURE OF A SUBJECT**
VORRICHTUNG, SYSTEM UND COMPUTERPROGRAMMPRODUKT ZUR DETEKTION VON MUSKELKRÄMPFEN BEI EINER PERSON
DISPOSITIF, SYSTÈME ET PRODUIT PROGRAMME D'ORDINATEUR DE DÉTECTION DE CRISE MUSCULAIRE D'UN SUJET

(30) Priority: 31.03.2016 EP 16163223
(43) Date of publication of application: 06.02.2019
(73) Proprietor: Koninklijke Philips N.V., 5656 AE Eindhoven (NL)
(72) Inventor: BAGGEN, Constant, Paul, Marie, Jozef, 5656 AE Eindhoven (NL); NOLAN, Julian, Charles, 5656 AE Eindhoven (NL); LAWRENSON, Matthew, John, 5656 AE Eindhoven (NL)
(74) Representative: Kapoor, Pavan Puneet
(86) International application number: PCT/EP2017/057628
(87) International publication number: WO 2017/167930

(56) References cited:
- WO-A1-2005/094679
- WO-A1-2013/144670
- WO-A1-2014/113813
- US-A1- 2015 313 496

## Description

### FIELD OF THE INVENTION

The present invention relates to a device and a system according to claims 1 and 7 for detecting and, preferably, reducing muscle seizure of a subject, e.g. a patient, user or person. Further, the present invention relates to a computer program product according to claim 14 for generating a control signal for controlling a vibration unit.

### BACKGROUND OF THE INVENTION

Parkinson's disease (PD) is a degenerative disorder of the central nervous system mainly affecting the motor system, which progresses slowly in most people. PD affects movement as well as producing motor symptoms. The motor symptoms of PD result of the loss of dopamine-generating brain cells. There are mainly four primary motor symptoms which occur in PD patients: tremor, slowness of movement, postural instability and lack of facial expression. One of the most apparent and well-known symptoms is the tremor, where the patient's limb moves with a given frequency. A tremor is an involuntary muscle seizure. The frequency of PD muscle seizure is typically between 4 and 6 Hz. Muscle seizure usually occurs in the hands, but it can also appear in other parts of the body, including the arms, legs, jaw and/or face.

At the moment, there is still no possibility of a causal treatment of PD, so there is no cure for PD patients. But medications can provide relief from the symptoms. When medications are insufficient to control symptoms, surgery and deep brain stimulation can be of use. Hence, the quality of life of PD patients is still affected by the motor symptoms and the treatment effects, for example side effects, of the disease. This calls for the creation of alternative treatments to muscle seizure decreasing the quality of life of tremor patients.

WO 2014/113813 A1 discloses a method and system to stimulate a peripheral nerve to treat Parkinson tremor with a peripheral nerve stimulator. This stimulator can be either a noninvasive or an implanted stimulator. The stimulation may be triggered by an electrical, a mechanical, or a chemical treatment.

The main practical problems still reside in the fact that the peripheral nerve stimulator, which is non-invasive, has to be attached to the patient's body over time regardless of whether it is useful. A muscle seizure and additionally the treatment of such tremor is thus rather painful and unpleasant for the subject.

The document WO2013/144670 A1 discloses eye tracking and detection of a user moving a user device in the context of tremor detection.

### SUMMARY OF THE INVENTION

It is an object of the present invention to provide a device and a system according to claims 1 and 7 which enable detection and, preferably, reduction of muscle seizure of a subject in a more comfortable and noninvasive manner, while being user-friendly, to further increase the quality of life for PD patients.

It is a further object of the present invention to provide a computer program product according to claim 14 for generating a control signal for controlling a vibration unit, which may be used for reduction of muscle seizure. The invention is defined by the appended claims.

In a first aspect of the present invention a device for detecting muscle seizure of a subject is presented comprising an activity input configured to obtain activity information related to a subject's activity when using a user device, a gaze input configured to obtain gaze information related to the subject's gaze when using the user device, a detection unit configured to detect a muscle seizure of the subject when using the user device by determining if the activity information indicates a reduction in the subject's activity using a user device and if the gaze information indicates that the subject's gaze is directed to the user device, and a control unit configured to generate a control signal, if the detection unit detects a muscle seizure of the subject configured to control a vibration unit attached to the subject and/or the user device to vibrate.

In a second aspect of the present invention a system for generating a control signal for controlling a vibration unit is presented comprising an activity acquisition unit configured to acquire activity information related to a subject's activity when using a user device, a gaze information acquisition unit configured to acquire gaze information related to the subject's gaze when using the user device, a device for detecting muscle seizure of a subject based on the acquired activity information and the acquired gaze information, and a vibration unit configured to vibrate in response to a control signal, generated by the device.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The inventors have found that when the activity of a subject is getting slower or even pauses/stops while using a user device, however the subject is still looking at the user device (or part of the user device), it is quite likely (i.e. it is interpreted as an indication) that the subject wishes to continue the activity but is restricted or even unable to do so due to muscle seizure. In order to make it possible for the subject to continue using the user device, the muscle seizure is thus treated by vibration in an effort to reduce or completely remove it. Hence, the detection of the muscle seizure itself is most comfortable and mostly inconspicuous for the subject. While using the user device, the subject is not interrupted or disturbed in his activity by the detection if a muscle seizure is present.

As used herein "detection of muscle seizure" may be understood as likelihood based on determining activity and gaze information. The muscle seizure is thus indirectly detected by the assessed likelihood.

"Reducing muscle seizure" shall be understood such that, when a vibration stimulus is triggered to the subject's tremor, the seizure (e.g. the duration and/or strength) may decrease to that the subject can continue with the activity.

"User device" may generally be understood as a device or a part of the device used by the subject in his activity.

"Activity information" may generally be understood as information indicating an activity, e.g. typing, writing, touching or just using the user device.

"Gaze information" may generally be understood as information about where the subject is looking at, i.e. the subject's eye attention.

"Control signal" means a signal configured to control or even activate the vibration unit to start vibrating.

The activity information can be acquired in different ways, e.g. with an algorithm that detects a reduction in activity, or potentially even a stop in activity, that may be attributed to reduction of hand mobility due to a muscle seizure. The activity information can be also acquired e.g. with an algorithm that assesses e.g. the text entered by the subject (e.g. user) and e.g. the touching of hyperlinks and determines the likelihood the user has stopped interacting with the user device. This algorithm may give a value, e.g. a numeric value, indicating the likelihood that the user has stopped user input or data entry. The activity information can be also acquired e.g. with an algorithm used to detect the likelihood that the user has paused his interaction with the user device. The gaze information can be acquired in different ways, e.g. with an algorithm that takes gaze location as an input and determines the likelihood that e.g. a given web page, email, etc. has been viewed to the extent the user would typically view it.

In a preferable embodiment, the detection unit is configured to determine, if the reduction in activity is above a predetermined threshold and/or a subject-related threshold and/or an activity-related threshold. This embodiment advantageously enables a more reliable prediction about the activity itself and its reduction due to the use of thresholds. This is based on the assumption that each subject as well as each different activity may have an individual activity behavior and different handling and therefore an individual threshold may be useful. This embodiment is further advantageous since the value of the thresholds is set in such a way that below this threshold the reduction of activity is unlikely due to a muscle seizure.

In a preferable embodiment, the detection unit is configured to determine if the activity is completely stopped or the activity level is below a predetermined activity level threshold and/or a subject-related activity level threshold and/or an activity-related activity level threshold. This embodiment enables a more reliable prediction about which kind of reduced activity is presented, e.g. either a stop, a pause or a deceleration. This is based on the assumption that generally each kind of activity is dependent on each different subject and each different activity. This embodiment is further advantageous since the value of the thresholds is set in such a way that below this threshold it is unlikely that the activity of the subject is stopped.

In another preferable embodiment, the detection unit is configured to determine if the time of the subject's gaze being directed to the user device exceeds a predetermined gaze threshold and/or a subject-related gaze threshold and or an activity-related gaze threshold. This embodiment enables a more reliable prediction if the subject is still looking at the user device and likely wishes to continue but is restricted to do so, particularly due to muscle seizure. The values of the thresholds may be set in such a way as to ensure this prediction.

In a preferable embodiment, the device further comprising a sensor input configured to obtain sensor information related to holding orientation and/or holding position of the user device and which subject's limb is using the user device, wherein the control unit is configured to control one or more vibration elements of the vibration unit based on the sensor information. This embodiment advantageously enables to control a specific vibration unit in order to achieve the best results in reduction of the muscle seizure.

In a preferable embodiment, the device further comprises a force input configured to obtain force information of subject's force input indicating how the user device is used by the subject, wherein the detection unit is further configured to determine if the force information indicates a muscle seizure. This embodiment is advantageous, because a further information source is used to make a more reliable prediction about a possible muscle seizure.

In a preferable embodiment of the proposed system, the vibration unit comprises two or more vibration elements. This embodiment advantageously enables a more precise reduction of the muscle seizure. With more than one vibration unit it is possible to decide which vibration unit is the most preferable one to achieve the best results in reduction of the muscle seizure.

In another preferable embodiment, the system further comprises a sensor unit configured to measure the holding orientation and/or holding position of the user device. This embodiment advantageously enables a judgment which vibration unit is likely the most effective one. The holding orientation and/or holding position information can be acquired in different ways, e.g. with a holding position algorithm used to detect the manner in which the user is holding the user device.

In a preferable embodiment, the activity acquisition unit is configured to detect starting, reducing, pausing and/or stopping activity information related to the subject's activity from the interaction between the subject and the user device. This embodiment advantageously enables estimating whether and how the subject is using the user device and drawing conclusions if a current task is completed by the subject or the subject is being interrupted due to a muscle seizure. This can be achieved by taking the time into account that the subject typically uses for an interaction with the user device and checking if the interaction time is increased due to the muscle seizure.

In a preferable embodiment, the system is implemented as a programmable electronic device, in particular a computer, a laptop, a mobile phone, a computing system comprised of a cluster of processors, a smart mobile device, a smartphone, a tablet device, personal digital assistant, a personal entertainment device, a smart watch, or a bracelet.

The activity acquisition unit may comprise a programmable user interface, a keyboard, a touchscreen, a mouse, remote control, a camera, and/or a joystick.

In a preferable embodiment, the gaze information acquisition unit is configured to detect subject's pupil movement, and/or eye movement, and/or head movement. This embodiment advantageously enables estimating whether the subject is still looking at the user device and if the user is looking at the user device for a typical time duration.

In a preferable embodiment, the gaze information acquisition unit comprises a programmable electronic device, a camera, and/or a smart eye wear device, in particular Google glasses.

Additionally to the method described above, a method for detecting muscle seizure of a subject is also foreseen by the present description, said method comprising: i) obtaining activity information related to a subject's activity when using a user device, ii) obtaining gaze information related to the subject's gaze when using the user device, iii) detecting a muscle seizure of the subject when using the user device by determining if the activity information indicates a reduction in the subject's activity using a user device and if the gaze information indicates that the subject's gaze is directed to said user device; and iv) generating a control signal, depending on detecting, controlling a vibration unit attached to the subject and/or the user device to vibrate.

These and other aspects of the invention will be apparent from and elucidated with reference to the embodiment(s) described hereinafter
It will be appreciated by those skilled in the art that two or more of the above-mentioned options, implementations, and/or aspects of the invention may be combined in any way deemed useful.

### BRIEF DESCRIPTION OF THE DRAWINGS

. In the following drawings
Fig. 1 shows a schematic diagram of a first embodiment of a system and device in accordance with the present invention;
Fig. 2 shows a schematic diagram of a second embodiment of a system and device in accordance with the present invention;
Fig. 3 shows a schematic diagram of a third embodiment of a system and device in accordance with the present invention;
Fig. 4a shows a fourth embodiment of a device in accordance with the present invention in the form of a smartphone in a typical user scenario;
Fig. 4b shows a schematic diagram of a method in accordance with the present invention using the smartphone shown in Fig 4a.
Fig. 5 shows a fifth embodiment of a device in accordance with the present invention in the form of a laptop in a typical user scenario;
Fig. 6 shows a sixth embodiment of a device in accordance with the present invention in the form of a smart watch in a typical user scenario; and
Fig. 7 shows a seventh embodiment of a device in accordance with the present invention in the form of a personal entertainment device in a typical user scenario.

### DETAILED DESCRIPTION OF THE INVENTION

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

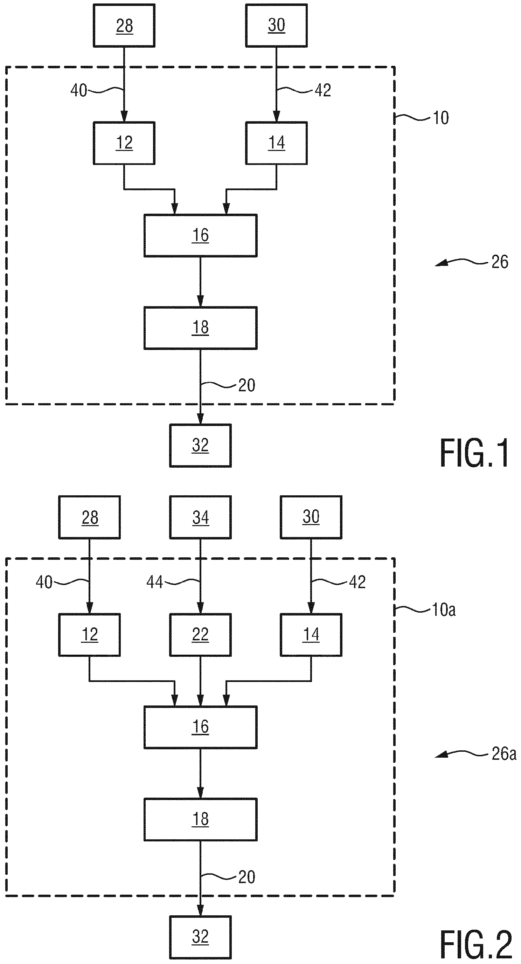

Fig. 1 shows a schematic diagram of a first embodiment of a system 26 and device 10 in accordance with the present invention. The system 26 shown in Fig. 1 comprises a device 10 for detecting and, preferably, reducing muscle seizure of a subject. Besides the device 10 the system 26 further comprises two acquisition units 28, 30 for acquiring activity information 40 and gaze information 42. The system 26 further comprises a vibration unit 32 for vibrating in response to a control signal 20 generated by the device 10, in particular to reduce muscle seizure of a subject.

The device 10 comprises an activity input 12 for obtaining activity information 40 related to a subject's activity when using a user device as acquired by an activity acquisition unit 28 which is part of the system 26. The function is to obtain (i.e. receive or retrieve) activity information 40, process the information with different algorithms, and pass the analyzed data to the detection unit 16.

The device 10 further comprises a gaze input 14 for obtaining gaze information 42 related to the subject's gaze when using the user device as acquired by a gaze information acquisition unit 30 which is part of the system 26. The function is to obtain (i.e. receive or retrieve) gaze information 42, process the information with different algorithms and pass the analyzed data to the detection unit 16.

The device 10 further comprises a detection unit 16 for detecting a muscle seizure of the subject 36 by determining if the activity information 40 indicates a reduction in the subject's activity using a user device and if the gaze information 42 indicates that the subject's gaze is directed to the user device.

The device 10 further comprises a control unit 18 for generating a control signal 20, if the detection unit 16 detects a muscle seizure of the subject 36 for controlling a vibration unit 32 attached to the subject 36 to vibrate, in particular for reducing the detected muscle seizure.

The system 26 further comprises a vibration unit 32. This vibration unit 32 preferably comprises two or more vibration elements. These vibration elements may be attached at different limbs of the subject, especially limbs which are used to operate the user device. Optionally, the vibration unit and its elements may be part of the user device and be contacted directly or indirectly to the subject's skin. The preferred vibration frequency and the duration of vibration may be predetermined and/or subject-related or may even be controlled individually dependent e.g. on the extent of muscle seizure. The vibration stimulus may e.g. start at low frequency and increase with time up to an upper limit.

The activity acquisition unit 28 is preferably configured to detect starting, reducing, pausing and/or stopping activity information 40 related to subject's activity from the interaction between the subject and the user device. Preferably, the activity acquisition unit 28 acquires each kind of activity from the subject applied to the user device over time. This provides to the possibility of determination of a most convenient value of a threshold which may be subject-related and/or activity related. Such threshold is preferably used to determine a reliable prediction about the activity itself and especially to which extent the activity is reduced (e.g. a stop, a pause or a deceleration). Further, the threshold is preferably used by the detection unit 16 to estimate if the reduction in activity is either above or below this threshold. Setting a suitable threshold value might be useful based on the assumption that generally each different kind of activity is dependent on each different subject and each different activity. In a preferable embodiment, the activity acquisition unit 28 comprises a programmable user interface, a keyboard, a touchscreen, a mouse, remote control, a camera, and/or a joystick.

The gaze information acquisition unit 30 may e.g. be configured to detect the subject's pupil movement, and/or eye movement, and/or head movement. With this information it is possible to set a most suitable value of the predetermined gaze threshold. Preferably, the acquiring of the gaze information 42 is recorded and processed over time. This provides to the possibility of determining a most convenient value of the activity-related gaze threshold. The acquiring may further be independent if the subject is interacting with the user device. This gives the opportunity to determine a value of the subject related gaze threshold. In a preferable embodiment, the gaze information acquisition unit 30 comprises a programmable electronic device, a camera, and/or a smart eye wear device, in particular Google glasses.

In a preferable embodiment, a further function of the detection unit 16 might be the detection if it is likely a subject has paused or slowed down in his activity due to muscle seizure in his device operating limb.

In a preferable embodiment, a further function of the control unit 18 might be the estimation which vibration element of the vibration unit is likely the most effective one.

In a preferable embodiment, the system 26 is implemented as a programmable electronic device, in particular a computer, a laptop, a mobile phone, a computing system comprised of a cluster of processors, a smart mobile device, a smartphone, a tablet device, personal digital assistant, a personal entertainment device, a smart watch, or a bracelet, as will be illustrated below.

The detection of muscle seizure is based on obtaining activity 40 and gaze information 42 and processing thus with algorithms, these algorithms can run parallel or sequentially. In a preferable embodiment they are used to estimate if a subject is getting slower or even pauses/stops, while using a user device, and if it is quite likely that the subject wishes to continue the activity but is restricted to do so due to muscle seizure. In detail the algorithms may be used to estimate if a current task is completed and if the subject is looking at the user device. When a muscle seizure is detected, algorithms may be used to decide which resources (e.g. vibration element) are available to provide vibrational stimuli to the subject's limb (e.g. hand/arm) operating with the user device, and also which of these vibration sources are likely to be most effective. The algorithms further might be used to generate a control signal 20 to apply a vibrational stimulus to the subject's limb.

Fig. 2 shows a schematic diagram of a second embodiment of a system 26a and device 10a. In addition to the element of the first embodiment, the system 26a further comprises a sensor unit 34 and a sensor input 22 for obtaining sensor information 44 from the sensor input 22. The sensor information 44 may be measured by motion and/or position sensors (e.g. accelerometer, gravity sensors, gyroscope, compass, rotational vector sensors, orientation sensors and/or magnetometers) which measure acceleration forces, rotational forces and the physical position of the user device. After assessing the orientation and/or holding position of the user device the control unit 18 controls the vibration elements of the vibration unit 32 by taking the sensor information 44 (information from 34) into account. In a preferable embodiment the vibration unit 32 comprises more than one vibration element, therefore the control unit 18 may have the additional function to determine which vibration element is the most preferable one to activate in order to achieve the best results in reduction of the muscle seizure.

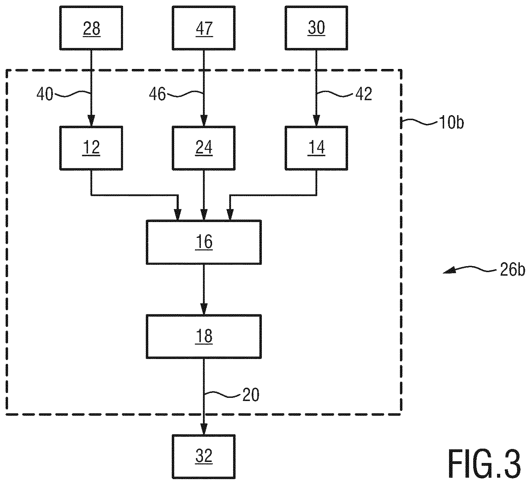

Fig. 3 shows a schematic diagram of a third embodiment of a system 26b and device 10b. In addition to the element of the first embodiment, the system 26b further comprises a force unit 47 and a force input 24 for obtaining force information 46 of subject's force input indicating how the user device is used by the subject. The detection unit 16 than determine if the force information 46, the activity information 40 and the gaze information 42 indicates a muscle seizure. Acquiring this information might be made by a force unit 47 e.g. a force touching sensor.

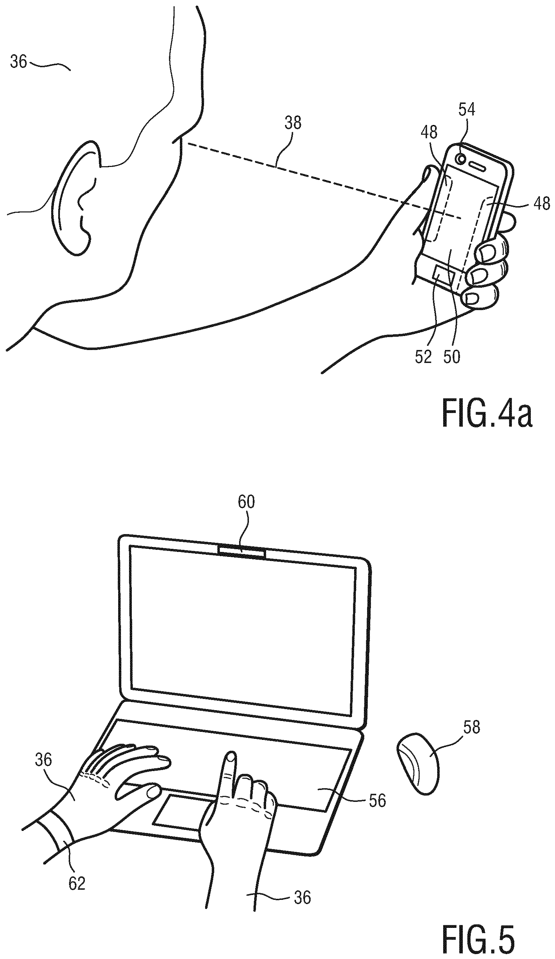

Fig. 4a shows a fourth embodiment of a device in the form of a smartphone in a typical user scenario. The smartphone is equal to the above mentioned user device. The user device is not the device in accordance with the present invention that is able to detect and, preferably, reduce muscle seizure. Optionally, the device could be a part of the user device.

The smartphone comprises processing units for obtaining and acquiring information about the subject's interaction with the smartphone. The smartphone further comprises a touchscreen 50, which is preferably configured for measuring the activity information. In a preferable embodiment the touchscreen 50 is able to measure the force applied on it by the subject. The smartphone further comprises a sensor unit 52 which may be capable of measuring the orientation and/or holding position of the smartphone, and/or which subject's limb is using the smartphone. The smartphone further comprises a camera 54, which records subject's gaze 38. The smartphone further comprises vibration elements 48, which can be arranged over the smartphone. In a preferable embodiment the smartphone comprises more than one vibration element 48.

The same or similar elements as in the smartphone might also be implemented in another smart mobile device, e.g. a tablet device or a personal digital assistant.

The smartphone further comprises a processing unit, where the information were analyzed and evaluate if a muscle seizure of a subject is detected and how to reduce these muscle seizure most efficacious. This processing unit may have the functions of the device 10 shown in Fig. 1. By use of one or more processing units it may be further able to execute the above described processing algorithms. In a preferable embodiment one or more of the following algorithms may be executed: an activity reduction algorithm, a likelihood of cessation of user input algorithm, a likelihood of activity completion algorithm, a likelihood of pause algorithm, a holding position algorithm, and a vibration unit selection algorithm.

In a preferable embodiment the vibration element 48 might not be arranged in the smartphone but being attached at the subject's limb e.g. integrated in a bracelet.

The activity reduction (AR) algorithm, may be an algorithm that detects a reduction in activity, or potentially even a stop in activity, that might be attributed to reduction of subject's limb mobility due to muscle seizure. The likelihood of cessation of user input (LCUI) algorithm might be an algorithm that assesses (i) the text being entered by the subject and/or (ii) the touching of hyperlinks and determines the likelihood the subject has stopped interacting with the user device. The likelihood of activity completion (LAC) algorithm, might be an algorithm that takes gaze location as an input and determines the likelihood a given web page, email etc. has been viewed to the extend the subject would typically view it. The likelihood of pause (LOP) algorithm might be an algorithm used to detect the likelihood the subject has pause his interaction with the user device. The holding position (HP) algorithm might be used to detect the manner in which the subject is holding the user device. The vibration unit selection (VUS) algorithm might be an algorithm used to select which vibration element(s) to activate.

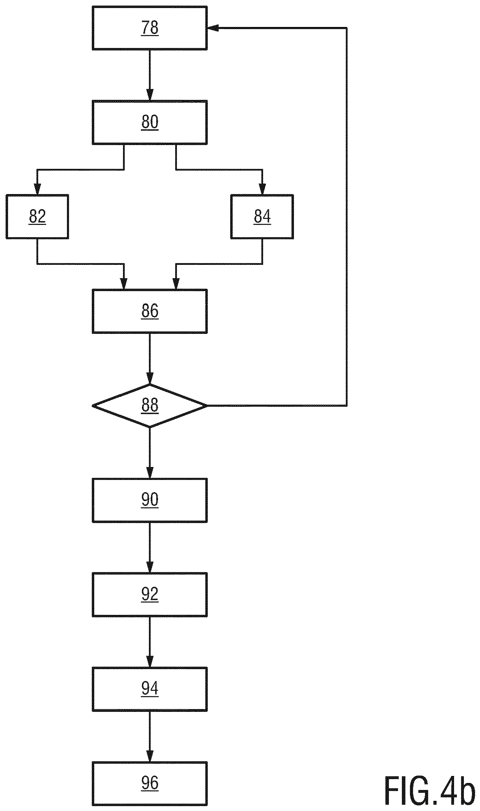

Fig. 4b shows a flow chart of a method according to the present invention which may be carried out by the smartphone shown in Fig 4a. When using the smartphone shown in Fig. 4a exemplary steps for detection and, preferably, reduction of muscle seizure will be explained in the following.

In a first step 78 the processing unit detects that the subject has begun to input information (for example the subject begins to write text, or touches a hyperlink).

In a second step 80 the AR algorithm senses for reductions in activity that may be attributed to the lack of mobility in the subject's hand. This might be done for example by measuring if the time between user interface interactions increases (e.g. the user types more slowly) and/or the force with which the subject touches the screen increases.

In a third step 82 the LCUI algorithm assesses in the following the likelihood of completion of the subject's input session and gives a LCUI value, may be a numeric value indicating the likelihood the subject has stopped user input data entry. This might be done for text entry for example by the following methods: First at the word level whether a word has been completed according to comparison to a dictionary and/or second the dictionary approach can be extended to set phrases, with the phrases also being in the dictionary and having either a likelihood that the subject will use the phrase and/or a level of completion that indicates that the phrase will be used, and/or third at the phrase/sentence level various grammatical probabilities can be applied, such as a sentence starts with a capital letter and ends with a full stop, and usually contains a subject and a predicate and/or finally at the document level some predictions of completion may be made if the subject performs a particular action, for example saves the document or sends an email. The likelihood of completion of the subject's input session for hyperlink on a web page entry might be done for example by the following method. The pattern of the subject's historical hyperlink touches may be assessed, and from this a probability calculated that the subject may have completed viewing the web page.

In a fourth step 84 simultaneously with the second/third step 80/82 the gaze information acquisition unit (e.g. a camera) might be activated and the gaze locations will be recorded. The location of the subject's gaze at any given time may be used in two ways: first as a direct input to the LOP algorithm and second as an input to the LAC algorithm, which then determines whether the current item being looked at (e.g. web page, email etc.) has been viewed to the extent the subject typically views such an item. The output of the algorithm might be the LAC value. This might be achieved first via a comparison to a lookup table that states the typical amount a web page or email etc. has been historically viewed by the subject, where 'amount' might be a duration of time, or proportion of material - for example whether the subject e.g. user typically views all of an email or article, or just the initial sections and second via a comparison to other users viewing the same material.

In a fifth step 86 the LOP algorithm takes the following inputs: LCUI Value, LAC Value, and the current gaze location. The algorithm might then determine the LOP Value, i.e. a numeric value indicating the likelihood the subject has either intentionally paused, or that the subject has not intentionally paused, but is unable to continue as their medical condition is preventing them from doing so. If in the sixth step 88 the LOP Value might be below a certain level (the "LOP threshold") the processing unit assumes the subject has validly paused, and waits for an indication the subject has resumed interaction with the user device. If this is the case the process might return to the first step 78. However the LOP value is above the LOP threshold the process move to the following step 90.

In the seventh step 90 the HP Algorithm takes either values ascertained from the device's inertial sensors and/or information from the device's touchscreen and might determine the likely holding orientation and position of the user device (comprising if being held by one or two hands, and if one hand, then which hand is being used).

In the eighth step 92 the user device polls available vibration elements. This might be done by the processing unit ascertains what vibration elements are available in the user device itself and/or the processing unit communicates with other devices that are paired with the user device and ascertains whether vibration elements are available on those devices.

In a preferable embodiment the processing unit also ascertains the location of the other devices. This might be done by querying a look-up table stored within the system, and/or an assessment of the measurements of inertial sensors on the other devices and/or the vibration elements are vibrated in turn and the effect of this motion assessed. Finally the processing unit might then have a list of all available vibration elements and their location.

In the ninth step 94 the VUS algorithm determines which vibration element on which device (or which combination of vibration elements) is most likely to vibrate the limb the subject is using to interface with the user device. Finally in the last step 96 the vibration element(s) chosen in the ninth step 94 vibrates.

Fig. 5 shows a fifth embodiment of a device in the form of a laptop in a typical user scenario. The laptop is equal to the above mentioned user device. The user device is not the device in accordance with the present invention that is able to detect and, preferably, reduce muscle seizure. Optionally, the device could be a part of the user device.

The laptop comprises a camera 60 for acquiring gaze information related to the subject's gaze. In a preferable embodiment the camera might not be installed in the laptop rather be flexible and/or transportable but might be able to communicate. The camera might to be installed in a manner that the subject's gaze can be possibly recorded. The laptop further comprises a keyboard 56 and optionally a mouse 58 with the function to acquire activity information from the subject 36, while interacting with the laptop. This might be either done by both the keyboard and the mouse, or might be done by one of them. The keyboard 56 and/or the mouse 58 may be further able to measure the force input of the subject while using the laptop. The laptop further comprises a processing unit, where the information from the camera 60 and the keyboard 56 and/or the mouse 58 were analyzed and were used to detect a muscle seizure as well for sending a control signal to the vibration unit 62 for reducing a muscle seizure. In a preferable embodiment the processing unit might not be a part of the laptop but a separate programmable device. Optionally the vibration unit 62 is part of a bracelet, shown in Fig. 5. In a preferable embodiment the vibration unit comprises more than one vibration elements which might be part of e.g. the keyboard 56 or the mouse 58. In a preferable embodiment the laptop might be a computer.

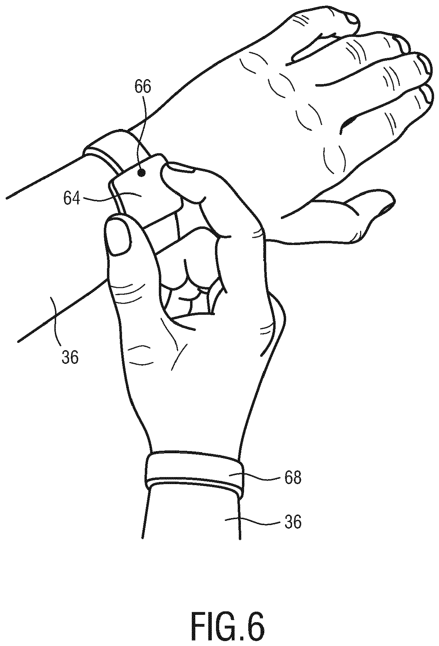

Fig. 6 shows a sixth embodiment of a device in the form of a smart watch in a typical user scenario. The smart watch comprises a camera 66 for recording the subject's gaze information. In a preferable embodiment the camera 66 might be not included in the smart watch, but be flexible and/or transportable but might be able to communicate. The camera might be integrated in e.g. glasses to obtain where the subject is looking at. The smart watch further comprises a touchscreen 64 to acquire the activity information. Optionally the touchscreen 64 might be a keyboard with buttons. The touchscreen 64 might be able to measure the force input which can indicate a muscle seizure. The smart watch further comprises a processing unit, where the information from the camera 66 and the touchscreen 64 were analyzed and were used to detect a muscle seizure. If a muscle seizure is detected the processing unit further be able to send a control signal to the vibration unit 68. In order to make the smart watch small and simple the processing unit may be not a part of the smart watch, either an additional programmable device. A vibration unit 68 might be fixed to the using limb, where the smart watch is not attached. The vibration unit 68 might be a part of a bracelet, which is able to communicate.

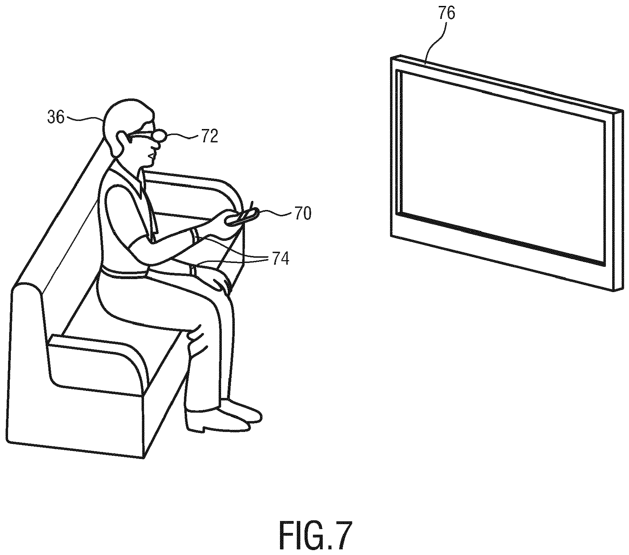

Fig. 7 shows a seventh embodiment of a device in the form of a personal entertainment device in a typical user scenario. The subject 36 wears glasses comprising a camera 72 (e.g. a Google glass), to acquire the subject's gaze, especially where the subject 36 is looking at. In an optional embodiment the camera 72 might be flexible and/or transportable but might be able to record the subject's gaze and further be able to communicate. With reference to Fig. 7 the subject 36 is using a remote control 70. The remote control could be also a different handheld device e.g. a joystick. The remote control 70 might be able to acquire activity information of the subject 36, by its keyboard. In a preferable embodiment the keyboard could also be a touchscreen or buttons on a joystick. The information of both the gaze and the activity might be processed by a processing unit, to generate a control signal for the vibration unit 74. This processing unit could be part of the personal entertainment device 76, part of the vibration unit 74, part of the remote control 70, part of the glasses 72 and/or be an additional programmable device, which is able to communicate. The vibration unit 74 might be a bracelet, which is preferably attached at the subject's interacting limb, but might be also attached on both arms. In a preferable embodiment the vibration unit 74 might be a part of the remote control 70 or a part of another handheld device.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

As discussed above, the processing unit, for instance a controller, implements the control method. The processing unit can be implemented in numerous ways, with software and/or hardware, to perform the various functions required. A processor is one example of a processing unit which employs one or more microprocessors that may be programmed using software (e.g., microcode) to perform the required functions. A processing unit may however be implemented with or without employing a processor, and also may be implemented as a combination of dedicated hardware to perform some functions and a processor (e.g., one or more programmed microprocessors and associated circuitry) to perform other functions.

Examples of processing unit components that may be employed in various embodiments of the present disclosure include, but are not limited to, conventional microprocessors, application specific integrated circuits (ASICs), and field-programmable gate arrays (FPGAs).

In various implementations, a processor or processing unit or a controller may be associated with one or more storage media such as volatile and non-volatile computer memory such as RAM, PROM, EPROM, and EEPROM. The storage media may be encoded with one or more programs that, when executed on one or more processors and/or controllers and/or processing units, perform at the required functions. Various storage media may be fixed within a processor or controller or processing unit or may be transportable, such that the one or more programs stored thereon can be loaded into a processor or controller or processing unit.

Any reference signs in the claims should not be construed as limiting the scope.

## Claims

1. A device (10) for detecting muscle seizure of a subject (36), said device (10) comprising:
- an activity input (12) configured to obtain activity information (40) related to a subject's activity when using a user device;
- a gaze input (14) configured to obtain gaze information (42) related to the subject's gaze when using the user device;
- a detection unit (16) configured to detect a muscle seizure of the subject (36) when using the user device by determining if the activity information (40) indicates a reduction in said subject's activity using a user device and if the gaze information (42) indicates that the subject's gaze is directed to said user device; and
- a control unit (18) configured to generating a control signal (20), if the detection unit (16) detects a muscle seizure of the subject (36), for controlling a vibration unit (32) attached to the subject (36) and/or the user device to vibrate.

2. The device (10) according to claim 1, wherein the detection unit (16) is configured to determine, if the reduction in activity is above a predetermined threshold and/or a subject-related threshold and/or an activity-related threshold.

3. The device (10) according to claim 1, wherein said detection unit (16) is configured to determine, if the activity is completely stopped or the activity level is below a predetermined activity level threshold and/or a subject-related activity level threshold and/or an activity-related activity level threshold.

4. The device (10) according to claim 1, wherein said detection unit (16) is configured to determine, if the time of the subject's gaze being directed to said user device exceeds a predetermined gaze threshold and/or a subject-related gaze threshold and or an activity-related gaze threshold.

5. The device (10) according to claim 1, further comprising a sensor input (22) configured to obtain sensor information (44) related to holding orientation and/or holding position of said user device and which subject's limb is using the user device, wherein said control unit (18) is configured to control one or more vibration elements of said vibration unit (32) based on said sensor information (44).

6. The device (10) according to claim 1, further comprising a force input (24) configured to obtain force information (46) of subject's force input indicating how the user device is used by the subject (36), wherein said detection unit (16) is further configured to determine if the force information (46) indicates a muscle seizure.

7. A system (26) for detecting muscle seizure of a subject (36), said system (26) comprising:
- an activity acquisition unit (28) configured to acquire activity information (40) related to a subject's activity when using a user device;
- a gaze information acquisition unit (30) configured to acquire gaze information (42) related to the subject's gaze when using the user device;
- a device (10) as claimed in claim 1 for detecting muscle seizure of a subject (36) based on the acquired activity information (40) and the acquired gaze information (42); and
- a vibration unit (32) configured to vibrate in response to a control signal (20), generated by the device (10).

8. The system (26) according to claim 7, wherein said vibration unit (32) comprises two or more vibration elements.

9. The system (26) according to claim 7, further comprising a sensor unit (34) configured to measure the holding orientation and/or holding position of said user device.

10. The system (26) according to claim 7, wherein said activity acquisition unit (28) is configured to detect starting, reducing, pausing and/or stopping activity information (40) related to said subject's activity from the interaction between the subject (36) and said user device.

11. The system (26) according to claim 7, implemented as a programmable electronic device, in particular a computer, a laptop, a mobile phone, a computing system comprised of a cluster of processors, a smart mobile device, a smartphone, a tablet device, personal digital assistant, a personal entertainment device, a smart watch, or a bracelet.

12. The system (26) according to claim 7, wherein the activity acquisition unit (28) comprises a programmable user interface, a keyboard, a touchscreen, a mouse, remote control, a camera, and/or a joystick.

13. The system (26) according to claim 7, wherein the gaze information acquisition unit (30) is configured to detect subject's pupil movement, and/or eye movement, and/or head movement and/or comprises a programmable electronic device, a camera, and/or a smart eye wear device, in particular Google glasses.

14. A computer program product comprising a computer readable medium having computer readable code embodied therein, the computer readable code being configured such that, on execution by a device according to claim 1, the device is caused to perform a method for detecting a muscle seizure and for generating a control signal for controlling a vibration unit, said method comprising:
- obtaining activity information (40) related to a subject's activity when using a user device;
- obtaining gaze information (42) related to the subject's gaze when using the user device;
- detecting a muscle seizure of the subject when using the user device by determining if the activity information (40) indicates a reduction in the subject's activity using the user device and if the gaze information (42) indicates that the subject's gaze is directed to said user device; and
- generating a control signal (20), if a muscle seizure of the subject is detected based on the activity information indicating a reduction in the subject's activity using the user device and the gaze information indicating that the subject's gaze is directed to said user device, for controlling a vibration unit (32) attached to the subject (36) and/or the user device to vibrate.

## Patentansprüche

1. Vorrichtung (10) zum Detektieren eines Muskelkrampfes eines Subjekts (36), wobei die Vorrichtung (10) Folgendes umfasst:
- einen Aktivitätseingang (12), der konfiguriert ist, um Aktivitätsinformationen (40) in Bezug auf die Aktivität eines Subjekts zu erhalten, wenn dieses eine Anwendervorrichtung verwendet;
- einen Blickeingang (14), der konfiguriert ist, um Blickinformationen (42) in Bezug auf den Blick des Subjekts zu erhalten, wenn dieses die Anwendervorrichtung verwendet,
- eine Detektionseinheit (16), die konfiguriert ist, um einen Muskelkrampf des Subjekts (36) zu detektieren, wenn dieses die Anwendervorrichtung verwendet, durch Bestimmen, ob die Aktivitätsinformationen (40) eine Verringerung der Aktivität des Subjekts, das eine Anwendervorrichtung verwendet, anzeigen und ob die Blickinformationen (42) anzeigen, dass der Blick des Subjekts auf die Anwendervorrichtung gerichtet ist; und
- eine Steuerungseinheit (18), die konfiguriert ist, um ein Steuerungssignal (20) zu erzeugen, wenn die Detektionseinheit (16) einen Muskelkrampf des Subjekts (36) detektiert, um zu steuern, dass eine Schwingungseinheit (32), die an dem Subjekt (36) und/oder der Anwendervorrichtung angebracht ist, schwingt.

2. Vorrichtung (10) nach Anspruch 1, wobei die Detektionseinheit (16) konfiguriert ist, um zu bestimmen, ob die Aktivitätsverringerung oberhalb eines vorgegebenen Grenzwerts und/oder eines subjektbezogenen Grenzwerts und/oder eines aktivitätsbezogenen Grenzwerts liegt.

3. Vorrichtung (10) nach Anspruch 1, wobei die Detektionseinheit (16) konfiguriert ist, um zu bestimmen, ob die Aktivität vollständig beendet ist oder das Aktivitätsniveau unterhalb eines vorgegebenen Aktivitätsniveau-Grenzwerts und/oder eines subjektbezogenen Aktivitätsniveau-Grenzwerts und/oder eines aktivitätsbezogenen Aktivitätsniveau-Grenzwerts liegt.

4. Vorrichtung (10) nach Anspruch 1, wobei die Detektionseinheit (16) konfiguriert ist, um zu bestimmen, ob die Zeit, die das Subjekt den Blick auf die Anwendervorrichtung richtet, einen vorgegebenen Blickgrenzwert und/oder einen subjektbezogenen Blickgrenzwert und/oder einen aktivitätsbezogenen Blickgrenzwert überschreitet.

5. Vorrichtung (10) nach Anspruch 1, weiter umfassend einen Sensoreingang (22), der konfiguriert ist, um Sensorinformationen (44) in Bezug auf die Halteausrichtung und/oder die Halteposition der Anwendervorrichtung und darauf, welche Gliedmaße des Subjekts die Anwendervorrichtung verwendet, zu erhalten, wobei die Steuerungseinheit (18) konfiguriert ist, um ein oder mehrere Schwingungselemente der Schwingungseinheit (32) basierend auf den Sensorinformationen (44) zu steuern.

6. Vorrichtung (10) nach Anspruch 1, weiter umfassend einen Krafteingang (24), der konfiguriert ist, um Kraftinformationen (46) des Subjekts zu erhalten, wobei der Krafteingang anzeigt, wie die Anwendervorrichtung von dem Subjekt (36) verwendet wird, wobei die Detektionseinheit (16) weiter konfiguriert ist, um zu bestimmen, ob die Kraftinformationen (46) einen Muskelkrampf anzeigen.

7. System (26) zum Detektieren eines Muskelkrampfes eines Subjekts (36), wobei das System (26) Folgendes umfasst:
- eine Aktivitätserfassungseinheit (28), die konfiguriert ist, um Aktivitätsinformationen (40) in Bezug auf die Aktivität eines Subjekts zu erhalten, wenn dieses eine Anwendervorrichtung verwendet;
- eine Blickinformationserfassungseinheit (30), die konfiguriert ist, um Blickinformationen (42) in Bezug auf den Blick des Subjekts zu erhalten, wenn dieses eine Anwendervorrichtung verwendet;
- eine Vorrichtung (10) nach Anspruch 1 zum Detektieren eines Muskelkrampfes eines Subjekts (36) basierend auf den erfassten Aktivitätsinformationen (40) und den erfassten Blickinformationen (42); und
- eine Schwingungseinheit (32), die konfiguriert ist, um in Reaktion auf ein von der Vorrichtung (10) erzeugtes Steuerungssignal (20) zu schwingen.

8. System (26) nach Anspruch 7, wobei die Schwingungseinheit (32) zwei oder mehr Schwingungselemente umfasst.

9. System (26) nach Anspruch 7, weiter umfassend eine Sensoreinheit (34), die konfiguriert ist, um die Halteausrichtung und/oder die Halteposition der Anwendervorrichtung zu messen.

10. System (26) nach Anspruch 7, wobei die Aktivitätserfassungseinheit (28) konfiguriert ist, um anhand der Interaktion zwischen dem Subjekt (36) und der Anwendervorrichtung Aktivitätsinformationen (40) in Bezug auf den Beginn, die Verringerung, die Pausierung und/oder die Beendigung der Aktivität des Subjekts zu detektieren.

11. System (26) nach Anspruch 7, implementiert als programmierbare elektronische Vorrichtung, insbesondere als Computer, Laptop, Mobiltelefon, Computersystem aus einer Gruppe von Prozessoren, intelligente mobile Vorrichtung, Smartphone, Tablet-Vorrichtung, persönlicher digitaler Assistent, persönliche Entertainment-Vorrichtung, Smartwatch oder Armband.

12. System (26) nach Anspruch 7, wobei die Aktivitätserfassungseinheit (28) eine programmierbare Benutzerschnittstelle, eine Tastatur, einen Touchscreen, eine Maus, eine Fernsteuerung, eine Kamera und/oder einen Joystick umfasst.

13. System (26) nach Anspruch 7, wobei die Blickinformationserfassungseinheit (30) konfiguriert ist, um eine Pupillenbewegung und/oder eine Augenbewegung und/oder eine Kopfbewegung des Subjekts zu detektieren, und/oder eine programmierbare elektronische Vorrichtung, eine Kamera und/oder eine Datenbrille, insbesondere Google Glass, umfasst.

14. Computerprogrammprodukt, umfassend ein computerlesbares Medium mit einem darin enthaltenen computerlesbaren Code, wobei der computerlesbare Code so konfiguriert ist, dass bei Ausführung durch eine Vorrichtung nach Anspruch 1 die Vorrichtung veranlasst wird, ein Verfahren zum Detektieren eines Muskelkrampfes und zum Erzeugen eines Steuerungssignals zum Steuern einer Schwingungseinheit durchzuführen, wobei das Verfahren Folgendes umfasst:
- Erhalten von Aktivitätsinformationen (40) in Bezug auf die Aktivität eines Subjekts, wenn dieses die Anwendervorrichtung verwendet;
- Erhalten von Blickinformationen (42) in Bezug auf den Blick des Subjekts, wenn dieses die Anwendervorrichtung verwendet;
- Detektieren eines Muskelkrampfes des Subjekts, wenn dieses die Anwendervorrichtung verwendet, durch Bestimmen, ob die Aktivitätsinformationen (40) eine Verringerung der Aktivität des Subjekts, das die Anwendervorrichtung verwendet, anzeigen und ob die Blickinformationen (42) anzeigen, dass der Blick des Subjekts auf die Anwendervorrichtung gerichtet ist; und
- Erzeugen eines Steuerungssignals (20), wenn ein Muskelkrampf des Subjekts detektiert wird, basierend auf den Aktivitätsinformationen, die eine Verringerung der Aktivität des Subjekts, das die Anwendervorrichtung verwendet, anzeigen, und den Blickinformationen, die anzeigen, dass der Blick des Subjekts auf die Anwendervorrichtung gerichtet ist, um zu steuern, dass eine Schwingungseinheit (32), die an dem Subjekt (36) und/oder der Anwendervorrichtung angebracht ist, schwingt.

## Revendications

1. Dispositif (10) pour détecter une crampe musculaire d'un sujet (36), ledit dispositif (10) comprenant :
- une entrée d'activité (12) configurée pour obtenir des informations d'activité (40) relatives à l'activité d'un sujet lorsqu'il utilise un dispositif utilisateur ;
- une entrée de regard (14) configurée pour obtenir des informations de regard (42) relatives au regard du sujet lorsqu'il utilise le dispositif utilisateur ;
- une unité de détection (16) configurée pour détecter une crampe musculaire du sujet (36) lorsqu'il utilise le dispositif utilisateur en déterminant si les informations d'activité (40) indiquent une réduction dans ladite activité du sujet en utilisant un dispositif utilisateur et si les informations de regard (42) indiquent que le regard du sujet est dirigé vers ledit dispositif utilisateur ; et
- une unité de commande (18) configurée pour générer un signal de commande (20), si l'unité de détection (16) détecte une crampe musculaire du sujet (36), pour commander à une unité de vibration (32) attachée au sujet (36) et/ou au dispositif utilisateur de vibrer.

2. Dispositif (10) selon la revendication 1, dans lequel l'unité de détection (16) est configurée pour déterminer si la réduction d'activité est supérieure à un seuil prédéterminé et/ou à un seuil lié au sujet et/ou à un seuil lié à l'activité.

3. Dispositif (10) selon la revendication 1, dans lequel ladite unité de détection (16) est configurée pour déterminer si l'activité est complètement arrêtée ou si le niveau d'activité est inférieur à un seuil de niveau d'activité prédéterminé et/ou à un seuil de niveau d'activité lié au sujet et/ou à un seuil de niveau d'activité lié à l'activité.

4. Dispositif (10) selon la revendication 1, dans lequel ladite unité de détection (16) est configurée pour déterminer si le temps du regard du sujet étant dirigé vers ledit dispositif utilisateur dépasse un seuil de regard prédéterminé et/ou un seuil de regard lié au sujet et/ou un seuil de regard lié à l'activité.

5. Dispositif (10) selon la revendication 1, comprenant en outre une entrée de capteur (22) configurée pour obtenir des informations de capteur (44) relatives à une orientation de maintien et/ou une position de maintien dudit dispositif utilisateur et quel membre du sujet utilise le dispositif utilisateur, dans lequel ladite unité de commande (18) est configurée pour commander un ou plusieurs éléments de vibration de ladite unité de vibration (32) sur la base desdites informations de capteur (44).

6. Dispositif (10) selon la revendication 1, comprenant en outre une entrée de force (24) configurée pour obtenir des informations de force (46) d'une entrée de force du sujet indiquant comment le dispositif utilisateur est utilisé par le sujet (36), dans lequel ladite unité de détection (16) est en outre configurée pour déterminer si les informations de force (46) indiquent une crampe musculaire.

7. Système (26) pour détecter une crampe musculaire d'un sujet (36), ledit système (26) comprenant :
- une unité d'obtention d'activité (28) configurée pour obtenir des informations d'activité (40) relatives à l'activité d'un sujet lorsqu'il utilise un dispositif utilisateur ;
- une unité d'obtention d'informations de regard (30) configurée pour obtenir des informations de regard (42) relatives au regard du sujet lorsqu'il utilise le dispositif utilisateur ;
- un dispositif (10) selon la revendication 1 pour détecter une crampe musculaire d'un sujet (36) sur la base des informations d'activité obtenues (40) et des informations de regard obtenues (42) ; et
- une unité de vibration (32) configurée pour vibrer en réponse à un signal de commande (20), généré par le dispositif (10).

8. Système (26) selon la revendication 7, dans lequel ladite unité de vibration (32) comprend deux ou plusieurs éléments de vibration.

9. Système (26) selon la revendication 7, comprenant en outre une unité de capteur (34) configurée pour mesurer l'orientation de maintien et/ou la position de maintien dudit dispositif utilisateur.

10. Système (26) selon la revendication 7, dans lequel ladite unité d'obtention d'activité (28) est configurée pour détecter des informations de démarrage, de réduction, de mise en pause et/ou d'arrêt d'activité (40) relatives à ladite activité du sujet depuis l'interaction entre le sujet (36) et ledit dispositif utilisateur.

11. Système (26) selon la revendication 7, mis en œuvre comme dispositif électronique programmable, en particulier un ordinateur, un ordinateur portable, un téléphone portable, un système informatique composé d'un ensemble de processeurs, un dispositif mobile intelligent, un téléphone intelligent, une tablette, un assistant numérique personnel, un dispositif de divertissement personnel, une montre intelligente, ou un bracelet.

12. Système (26) selon la revendication 7, dans lequel l'unité d'obtention d'activité (28) comprend une interface utilisateur programmable, un clavier, un écran tactile, une souris, une télécommande, une caméra, et/ou une manette.

13. Système (26) selon la revendication 7, dans lequel l'unité d'obtention d'informations de regard (30) est configurée pour détecter un mouvement de pupille, et/ou un mouvement d'oeil, et/ou un mouvement de tête du sujet, et/ou comprend un dispositif électronique programmable, une caméra, et/ou un dispositif de lunettes intelligentes, en particulier des lunettes Google.

14. Produit de programme informatique comprenant un support lisible par ordinateur présentant un code lisible par ordinateur incorporé dans celui-ci, le code lisible par ordinateur étant configuré de sorte que, lors de l'exécution par un dispositif selon la revendication 1, le dispositif est amené à effectuer un procédé pour détecter une crampe musculaire et pour générer un signal de commande pour commander une unité de vibration, ledit procédé comprenant les étapes consistant à :
- obtenir des informations d'activité (40) relatives à l'activité d'un sujet lorsqu'il utilise un dispositif utilisateur ;
- obtenir des informations de regard (42) relatives au regard du sujet lorsqu'il utilise le dispositif utilisateur ;
- détecter une crampe musculaire du sujet lorsqu'il utilise le dispositif utilisateur en déterminant si les informations d'activité (40) indiquent une réduction dans l'activité du sujet en utilisant le dispositif utilisateur et si les informations de regard (42) indiquent que le regard du sujet est dirigé vers ledit dispositif utilisateur ; et
- générer un signal de commande (20), si une crampe musculaire du sujet est détectée sur la base des informations d'activité indiquant une réduction dans l'activité du sujet en utilisant le dispositif utilisateur et des informations de regard indiquant que le regard du sujet est dirigé vers ledit dispositif utilisateur, pour commander à une unité de vibration (32) attachée au sujet (36) et/ou au dispositif utilisateur de vibrer.
